# FASCICULE DE BREVET EUROPEEN

(11) **EP 0 681 470 B1**
(45) Date de publication et mention de la délivrance du brevet: **17.07.1996**
(21) Numéro de dépôt: 94905751.7
(22) Date de dépôt: 28.01.1994
(51) Int. Cl.: A61K 9/107, A61K 39/39, A61K 39/245, A61K 39/21

(54) **COMPOSITION DE VACCIN SOUS UNITAIRE RECOMBINANT VIVANT ET PROCEDE DE PREPARATION**
ZUSAMMENSETZUNG BESTEHEND AUS EINEM REKOMBINANTEN LEBENDEN UNTEREINHEITSIMPFSTOFF UND VERFAHREN ZUR HERSTELLUNG
RECOMBINANT LIVING SUBUNIT VACCINE COMPOSITION AND PROCESS FOR ITS PREPARATION

(30) Priorité: 29.01.1993 FR 9300942
(43) Date de publication de la demande: 15.11.1995
(73) Titulaire: S.E.P.P.I.C., SOCIETE D'EXPLOITATION DE PRODUITS POUR LES INDUSTRIES CHIMIQUES, F-75321 Paris Cédex 07 (FR)
(72) Inventeur: GANNE, Vincent, F-94210 La-Varenne-Saint-Hilaire (FR)
(74) Mandataire: Caen, Thierry Alain
(86) Numéro de dépôt international: FR9400106
(87) Numéro de publication internationale: WO9416681

(56) Documents cités:
- EP-A- 0 129 923
- WO-A-91/00107
- WO-A-92/11028
- NATURE. vol. 339 , 1 Juin 1989 , LONDON GB pages 385 - 388 D.J. EVANS ET AL. 'AN ENGINEERED POLIOVIRUS CHIMAERA ELICITS BROADLY REACTIVE HIV-1 NEUTRALIZING ANTIBODIES.'
- JOURNAL OF GENERAL VIROLOGY vol. 71, no. 10 , Octobre 1990 , READING, GB pages 2425 - 2431 M. ELOIT ET AL. 'CONSTRUCTION OF A DEFECTIVE ADENOVIRUS VECTOR EXPRESSING THE PSEUDORABIES VIRUS GLYCOPROTEIN gp50 AND ITS USE AS A LIVE VACCINE.' cité dans la demande
- A. ADAM 'SYNTHETIC ADJUVANTS' 1985 , JOHN WILEY & SONS , NEW YORK, N.Y., US
- VACCINE vol. 10, no. 4 , 1992 , GUILDFORD GB pages 226 - 230 H.P.A. HUGHES ET AL. 'MULTIPLE ADMINISTRATION WITH INTERLEUKIN-2 POTENTIATES ANTIGEN-SPECIFIC RESPONSES TO SUBUNIT VACCINATION WITH BOVINE HERPESVIRUS-1 GLYCOPROTEIN IV.'

## Description

La présente invention concerne de nouvelles compositions vaccinantes associant des vaccins sous unitaires recombinants vivants associés à des émulsions injectables, notamment du type eau dans huile (E/H), huile dans eau (H/E) ou eau dans huile dans eau (E/H/E), pour l'administration en médecine vétérinaire ou humaine.

La présente invention concerne plus particulièrement les vaccins vivants recombinants dans lesquels la protéine recombinante, qui est une protéine recombinante impliquée dans la réponse immunitaire protectrice contre un microorganisme, en particulier contre un virus enveloppé, est exprimée in vivo par un vecteur viral constitué par un virus non enveloppé tel l'adénovirus, le virus de la vaccine, le Canarypox virus ou les baculovirus.

La vaccination par des microorganismes vivants présente de nombreux avantages bien connus comparés à la vaccination par des vaccins non vivants constitués de micro-organismes tués ou de protéines ou peptides isolés.

Parmi les vaccins vivants, les vaccins vivants atténués sont largement utilisés, mais ils ont comme inconvénient de présenter des risques pathogènes ; ainsi, il existe toujours une possibilité d'une part, d'intégration de l'acide nucléique viral dans le génome de l'hôte, et d'autre part, de réversion vers une forme virulente.

Par ailleurs, les mutations ou délétions conduisant à l'atténuation de la virulence des vaccins vivants atténués entrainent parfois une diminution de la réponse immunitaire, nécessitant l'injection de doses importantes de composition vaccinale. L'utilisation d'adjuvants a été testé afin de combler la diminution de la réponse immunitaire.

Ainsi le brevet EP 129923 utilise des adjuvants huileux de type H/E pour améliorer la réponse immunitaire.

Si l'on constate ainsi une certaine amélioration de la réponse immunitaire, les microorganismes vivants atténués tels des virus vivants atténués, se présentant habituellement sous forme de lyophilisat, nécessite une solubilisation dans un milieu essentiellement aqueux. La quantité d'huile que l'on peut alors introduire dans la phase aqueuse est très limitée.

Il s'ensuit que seules des émulsions du type H/E peuvent être préparées à partir d'un vaccin vivant atténué.

Par ailleurs, certains microorganismes vivants atténués, tels les virus enveloppés vivants atténués, au contact direct d'une huile ou d'une phase huileuse, peuvent être inactivés, notamment sous l'effet d'une perte de l'intégrité de leur membrane biologique.

Les progrès du génie génétique permettent d'obtenir des vaccins sous unitaires recombinants, consistant en des antigènes de plus en plus purifiés, ou totalement synthétiques.

La pureté des vaccins sous unitaires recombinants est un atout incontestable sur le plan de la sécurité du vaccin comparativement aux vaccins atténués vivants.

Habituellement, les vaccins sous unitaires recombinants consistent en une ou plusieurs protéines purifiées produites par des micro-organismes recombinants et injectées chez l'hôte.

En raison de leur pureté même, ces vaccins sous unitaires recombinants non vivants, correspondant à ces vaccins constitués de protéines purifiées produites par des microorganismes recombinants, conduisent en général à une diminution de l'efficacité immunologique ; c'est pourquoi, l'emploi de vaccins sous unitaires recombinants nécessite d'utiliser une quantité importante d'antigènes.

De plus, les vaccins sous unitaires recombinants non vivants nécessitent plusieurs injections ce qui est difficilement compatible avec des impératifs économiques.

Plus récemment, ont été élaborés des vaccins sous unitaires recombinants vivants, consistant en un micro-organisme vivant, généralement un virus, en tant que vecteur recombinant et dans lequel est inséré un gène exogène. Selon l'invention, les vaccins sous-unitaires recombinants vivants sont directement introduits chez l'hôte, dans lequel la protéine est exprimée in vivo, par le gène exogène, inséré dans ledit microorganisme, pour déclencher la réaction immunitaire.

De tels vaccins ont été décrits dans l'article de M. Eloit et al., Journal of General Virology (1990), 71, 2425-2431 ou dans la demande internationale WO-A-91/00107. Cette dernière décrit plus précisément une méthode pour produire une réponse immune chez l'homme ou l'animal au moyen d'un tel vaccin sous unitaire recombinant vivant qu'il est toutefois nécessaire d'associer à un autre vaccin, consistant en un vecteur codant pour la HIV reverse transcriptase (HIVRT).

Ces deux vaccins sont injectés indépendamment l'un de l'autre, à plusieurs jours, voir plusieurs semaines d'intervalle. Le vaccin HIVRT peut être associé à un adjuvant tel une émulsion E/H, des sels minéraux, des polynucléotides ou des substances naturelles d'origine bactérienne. Le vaccin HIVRT est utile en tant que stimulant (booster) immunitaire du vaccin sous unitaire recombinant vivant.

L'utilisation de vaccins sous unitaires recombinants vivants permet de combler partiellement les inconvénients des vaccins sous unitaires recombinants non vivants ; il nécessite cependant d'injecter des micro-organismes recombinants tels des virus recombinants en des quantités et concentrations très importantes.

Mais même ainsi, les réponses immunitaires obtenues restent encore insatisfaisantes. Pour éviter ces inconvénients, comme préconisé dans ladite demande internationale WO-A-91.00107, il est considéré comme nécessaire d'injecter outre le vaccin sous unitaire recombinant, un autre vaccin jouant le rôle de stimulant (booster) immunitaire.

L'invention consiste alors en des vaccins sous unitaires recombinants vivants obviant aux inconvénients mentionnés ci-dessus.

De façon surprenante, l'invention permet en outre d'associer directement des huiles en quantité importante à des microorganismes vivants, tels des virus recombinants vivants, exprimant au moins un antigène d'un virus enveloppé.

La présente invention concerne une nouvelle composition de vaccin vivant recombinant caractérisé en ce qu'elle comprend au moins un vaccin sous unitaire recombinant vivant associée à une émulsion injectable comportant (i) au moins une phase aqueuse et (ii) une phase huileuse comprenant au moins une huile.

Plus particulièrement, les vaccins de l'invention sont des vaccins sous-unitaires recombinants vivants comprenant à titre de vecteur recombinant, un virus recombinant Avantageusement, ce virus est non enveloppé. Il peut notamment être choisi parmi les adénovirus, le virus de la vaccine, le virus Canarypox, les herpes virus, les baculovirus.

Les vaccins recombinants de l'invention sont également caractérisés par l'association avec une émulsion injectable à base d'huile et d'eau ,et le cas échéant d'un système émulgateur, d'un vecteur recombinant viral non enveloppé vivant dont le génome contient, insérée de préférence dans une partie non essentielle pour la réplication du virus enveloppé correspondant, une séquence codant pour une sous-unité antigénique induisant une synthèse d'anticorps et/ou un effet protecteur contre le susdit virus enveloppé ou micro-organisme pathogène. Les sous-unités antigéniques peuvent être par exemple, une protéine, une glycoprotéine, un peptide, ou une fraction peptidique immunogène et/ou protectrice contre une infection par un micro-organisme vivant tel un virus enveloppé, une bactérie ou un parasite.

Ladite émulsion peut être du type H/E, E/H ou E/H/E.

L'intéret de l'invention consiste dans la possibilité d'obtenir une composition stable, immunogène, fluide, dans une émulsion huileuse et permettant, de manière surprenante, de diminuer la quantité d'immunogènes par rapport à une composition aqueuse du même immunogène.

Les huiles, constituées de ladite phase huileuse, pouvant être utilisées dans la composition de vaccin selon l'invention sont avantageusement des huiles fluides choisies parmi les huiles minérales naturelles ou synthétiques, ou les huiles non minérales d'origines végétales, animales ou synthétiques, connues pour leur absence ou leur faible toxicité.

Elles doivent être liquides à la température de stockage (+4°C) ou au moins donner des émulsions liquides à cette température.

On choisira en particulier des huiles minérales à chaîne linéaire ayant un nombre d'atomes de carbone de préférence supérieur à 16 et exemptes de composés aromatiques.

Des exemples connus sont le MARCOL 52 (produit par ESSO France) et le DRAKEOL 6VR (produit par PENRECO USA).

On peut également utiliser des huiles synthétiques telles que les polyisobutènes ou les polyisoproprènes.

Parmi les huiles végétales, on choisira des huiles insaturées riche en acide oléique qui sont biodégradables, par exemple les huiles d'arachide, d'olive, de sésame, de soja, de germe de blé.

Pour les huiles animales, les mêmes critères de tolérance et d'efficacité immunologiques sont requis. A titre d'exemple d'huile animale, on peut citer, le squalène, le squalane, l'huile de spermaceti.

Un mélange des huiles citées précédemment est avantageusement utilisé dans le cadre de la présente invention.

Habituellement les compositions vaccinales selon l'invention comportent en outre un système émulgateur.

Il doit être adapté pour donner des préparations injectables de type E/H, H/E ,E/H/E fluides et stables.

Il se compose d'un ou plusieurs agents tensioactifs ayant en mélange un caractère lipophile ou hydrophile caractérisé par un nombre HLB (Hydrophile - Lipophile - Balance) compris entre 1 et 19.

Les émulgateurs sont de préférence des esters obtenus par condensation d'un acide gras liquide à 20°C sur un sucre (mannitol, glucose, saccharose) ou du glycérol ou des dérivés de ces esters.

Les acides gras saturés sont de préférence ceux ayant au moins 16 atomes de carbone et en particulier les acides oléique, linoléique, ricinoléique, cétostéarique.

On utilise de préférence les esters du mannitol ou des dérivés d'esters de mannitol. A titre d'ester de mannitol, on peut notamment citer, les oléates de mannitol obtenus, par anhydrisation de la chaîne carbonée polyhydroxylée du mannitol qui se cyclise en 1-4 ou en 2-6.

Lesdits dérivés d'ester peuvent consister en des esters dont l'hydrophilie est modifiée par greffage de fonctions hydrophiles telles qu'alcool, polyol, oxyde d'éthylène, oxyde de propylène, acide carboxylique, amine, amide.

Tous les émulgateurs utilisés doivent être pharmaceutiquement acceptables pour un usage en injectable, notamment être dépourvus de métaux lourds, et présenter des indices d'acide ou de peroxydes très faibles.

Il est également souhaitable qu'ils satisfassent les normes de tests d'innocuité tels que , par exemple, celui décrit par S.S. BERLIN (Annals of allergy, 1962, 20, 473).

Les émulgateurs utilisés forment avec l'huile choisie une phase huileuse, homogène, limpide et stable qui sera émulsionnée avec le milieu aqueux propre à chaque préparation injectable.

Le tableau 1 résume la nature minérale ou non minérale, c'est-à-dire métabolisable des huiles, le type d'émulsion vaccinale obtenu et les principales caractéristiques de ces huiles.

Les compositions de vaccin selon l'invention peuvent être utilisées pour préparer un vaccin destiné à vacciner l'homme ou l'animal, notamment les porcs, les ovins, les bovins, les amidés, les félidés, les volailles et les poissons.

Les concentrations de vaccins sous unitaires recombinants vivants dans les compositions selon l'invention peuvent varier en fonction de la nature de l'animal à vacciner et de celle dudit vaccin. Ces concentrations sont généralement comprises entre 10² et 10¹⁵ micro-organismes ou virus / ml et de préférence entre 10⁵ et 10¹² micro-organismes ou virus/ml.

Les volumes de la composition de vaccin selon l'invention à injecter à l'homme ou à l'animal, sont fonction du poids de celui-ci ainsi que de la nature dudit vaccin. Ces volumes peuvent être compris entre 10 µl et 5 ml. Pour l'homme ou un animal de poids voisin, tel certains primates, les volumes injectés peuvent être compris entre 0,25 et 2ml.

Habituellement, une composition de vaccin selon l'invention comprend moins de 1µg/ml, de préférence moins de 0,3 µg/ml d'antigène, exprimée par le gène exogène dudit vaccin sous-unitaire recombinant vivant, préalablement à l'injection de ladite composition. Selon l'invention, en effet, l'antigène déclenchant le phénomène immunitaire est essentiellement synthétisé in vivo par ledit vaccin ; soit après que celui-ci ait été injecté à l'homme ou à l'animal à vacciner.

Les compositions de vaccin selon l'invention peuvent être préparées par différents procédés classiques, par exemple selon les procédés décrits dans EP 480981 et EP 480982. Un tel procédé permettant l'obtention d'une composition selon l'invnetion peut consister à émulsionner un vaccin vivant recombinant en solution aqueuse avec une huile minérale ou non minérale ou un mélange d'huile minérale et non minérale sous la forme d'une émulsion E/H, H/E ou E/H/E. L'huile ou les huiles peuvent être associées à un adjuvant de l'immunité cellulaire telle l'avridine".

Les proportions pondérales de la phase huileuse contenant l'huile associée ou non au système émulgateur et de la phase aqueuse contenant le antigènes ou les principes actifs varient respectivement dans l'émulsion de 5 à 95% de phase huileuse, pour de 95 à 5% de phase aqueuse notamment de 25 à 75% de phase huileuse pour 75 à 25% de phase aqueuse.

Le mélange de la phase huileuse et de la phase aqueuse se fait de manière classique, sous agitation à des températures comprises entre 20° et 40°C. L'ensemble des préparations obtenues doivent être stables, c'est à dire que la préparation émulsionnée ne doit pas déphaser, ce qui signifie l'absence de libération de la phase antigénique ou de la phase huileuse dans les conditions de stockage normales pour ce type de produit. Les compositions selon l'invention peuvent présenter une stabilité supérieure ou égale à 12 mois à 4°C.

De plus, ces préparations vaccinales sont fluides, c'est à dire que la viscosité mesurée par un appareil à mobile tournant du type BROOKFIELD peut être inférieure à 200 mPa.s, avantageusement inférieure à 300 mPa.s à 25°C.

Le caractère huileux ou aqueux de la phase continue de l'émulsion est caractérisée par la conductivité mesurée en microsiemens par cm.

Pour des valeurs inférieures à 20 microsiemens par cm environ, on obtient une phase continue huileuse à température ambiante.

Font également parties de l'invention les vaccins recombinants vivants dans lequel la séquence codante dans ledit vaccin code par exemple pour une sous-unité antigénique des virus HIV tels les virus HIV1 ou HIV2, ou le virus FIV, l'expression de cette sous-unité antigénique induisant une synthèse d'anticorps et/ou un effet protecteur contre ces mêmes virus.

Dans les exemples qui suivent, la composition vaccinale utilisée est un adénovirus défectif exprimant la glycoprotéine gp 50 du virus de la pseudorage (PRV). La construction de l'adénovirus recombinant Ad-gp 50, l'expression de l'antigène gp 50 dans des cultures cellulaires, la réponse immunitaire et la protection conférée par vaccination de souris et de lapins avec Ad-gp 50 sont décrits dans la publication de M.Eloit et al, Journal of General Virology (1990), 71 : 2425-2431.

Brièvement, la lignée cellulaire 293, une lignée embryonnaire humaine transformée par un adénovirus de type 5 (Ad-5) (Graham et al, 1977, Journal of General Virology, 36 : 59-72) a été utilisée pour la transfection, la multiplication et le titrage de l'adénovirus.

La construction de Ad - gp 50 utilise un Ad 5 délété dans la région E3 non essentielle à la croissance du virus.

L'Ad - gp 50 exprime de hauts niveaux de gp 50 dans les lignées cellulaires complétant ou non E1A, et notamment dans les cellules Hela déplétées pour le gène E1A.

Les auteurs remarquent que, in vivo, la réponse en anticorps, mesurée par un test ELISA, est significative pour des concentrations élevées en Ad-gp50 tandis que la protection conférée à des souris ou des lapins est faible après épreuve à l'infection par le PRV.

L'intérêt de l'invention consiste à émulsionner ce type de vaccin vivant recombinant dans des huiles associées à des émulgateurs décrits plus haut afin d'augmenter, d'une part, leur pouvoir immunogène et protecteur en diminuant les doses de virus vivants injectés, et d'autre part, d'augmenter la synthèse de cytokines telles les Interleukines, en particulier les IL2 et les IL6.

Dans les différents exemples qui suivent le virus Aujeszky est équivalent à PRV.

### Exemple 1 (comparatif) : Induction d'anticorps et efficacité de la vaccination en absence d'adjuvants

Le vaccin est constitué d'adénovirus recombinants Ad - gp 50 décrits précédemment.

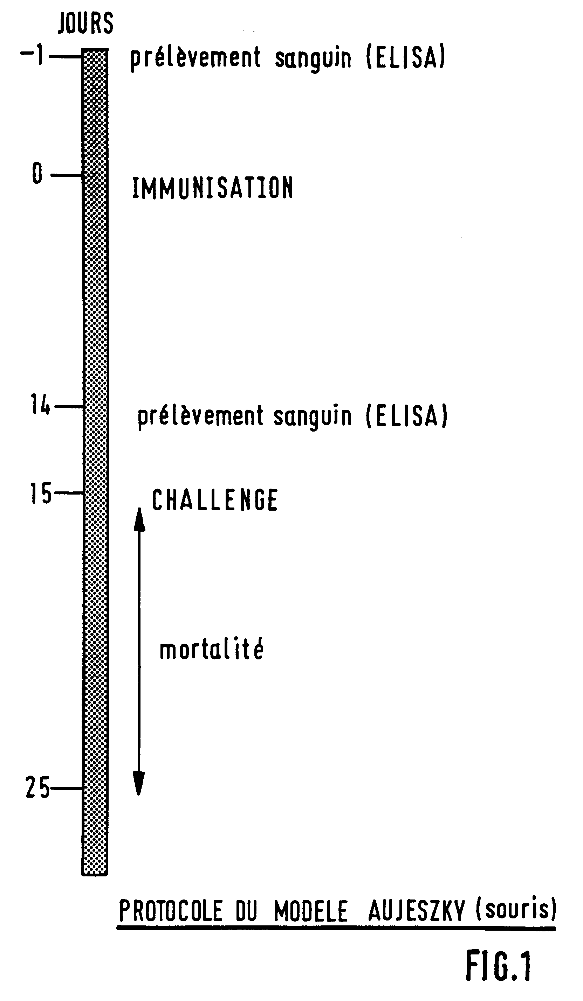

Les compositions de vaccins, à trois concentrations d'Ad-gp 50, C1, C2 et C3, sont inoculés à JO par voie sous cutanée dans des souris OF1, réparties en trois groupes, (10 souris par groupe), à raison de 100 µl par souris. Les trois concentrations vaccinales utilisées sont définies comme suit :
- 10⁹ virus/ml, soit 10⁸ TC ID50/souris (C1)
- 3.10⁸ virus/ml, soit 10^{7,4} TCID/50 souris (C2)
- 3.10⁷ virus/ml, soit 10^{6,4} TCID/50 souris (C3)
Le protocole utilisé pour évaluer l'efficacité de la préparation vaccinale est décrit sur la figure 1.

Un prélèvement de sérum est effectué deux semaines plus tard pour évaluer la quantité d'anticorps anti gp 50 induite par les préparations vaccinales.

Pour évaluer l'efficacité des vaccins, une épreuve "challenge" est effectuée en présence d'une préparation de virus Aujeszky virulente à la concentration de 20 DL50, définie par une expérimentation préliminaire.

Ladite concentration correspond à une mortalité de 100% chez des souris non vaccinées.

La mortalité est observé sur 240 heures ; au - delà de cette période toutes les souris résistent à l'épreuve.
Deux paramètres sont validés :
- le taux de survie, exprimé en pourcentage, observé à la fin de l'expérimentation
- la cinétique de mortalité.

### Résultats

### - Dosage des anticorps anti gp 50 :

Le prélèvement du sérum est effectué deux semaines après les immunisations.
Le dosage des anticorps anti gp 50 est effectué par ELISA, par la technique décrite par Eloit et coll., Veterinary Record, 1989, 124 : 91-94.

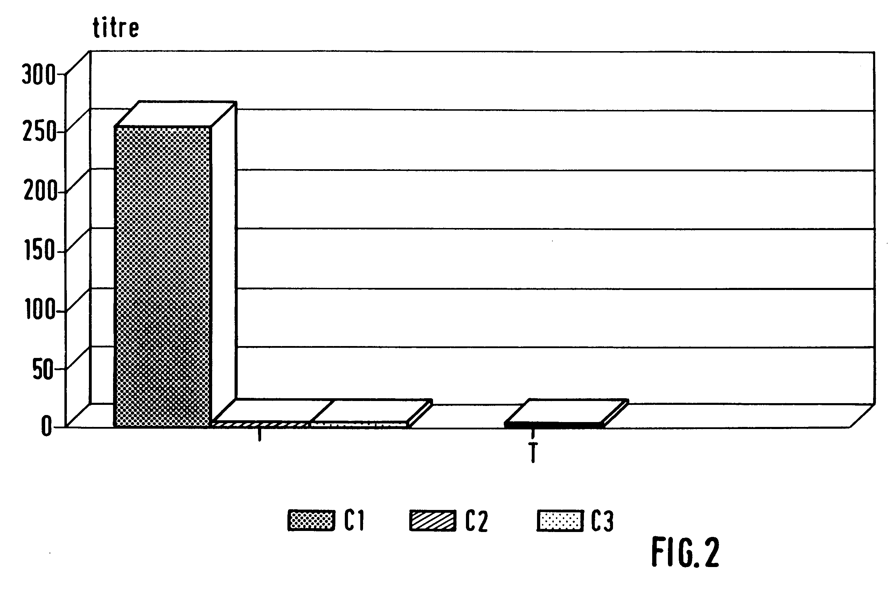

Les résultats obtenus sont représentés dans la figure 2, dans laquelle :
- C1, C2, C3 représentent respectivement les trois concentrations d'Ad-gp 50 : 10⁹, 3.10⁸ et 3.10⁷ virus/ml.
- les titres sont indiqués en ordonnées et indiquent la dilution nécessaire à l'obtention d'une D.O. (densité optique) supérieure au bruit de fond;
- T indique un témoin absolu : non vacciné ;

En absence d'adjuvant, une induction d'anticorps est obtenue uniquement à la concentration de 10⁹ Ad-gp50/ml ; aux concentration de 3.10⁸ et 3.10⁷ Ad-gp50/ml, on ne détecte pas d'anticorps anti gp 50.

### - Epreuve avec le virus virulent

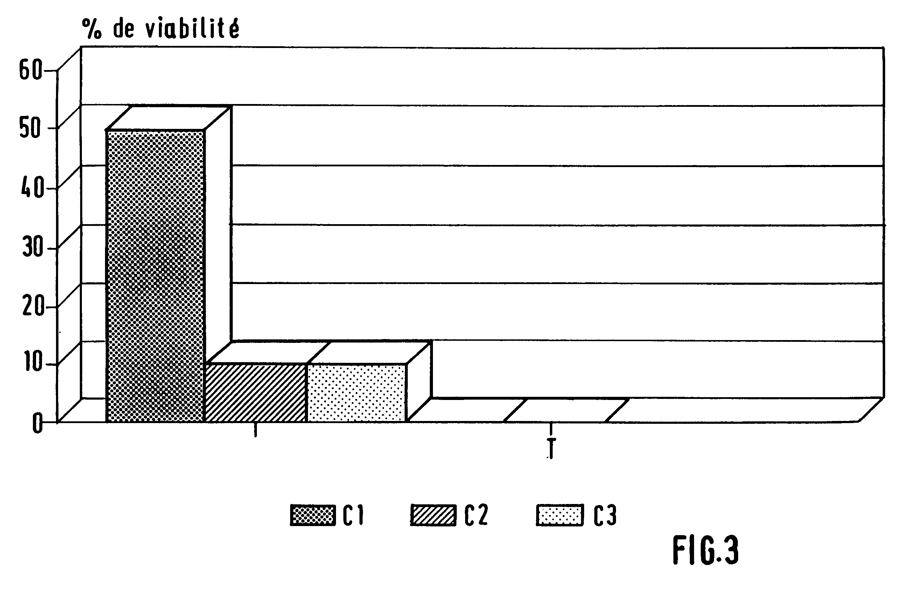

La résistance à l'épreuve par du virus Aujeszky est évaluée aux trois concentrations vaccinales C1, C2 et C3.
Les taux de survie après l'épreuve sont présentées sur la figure 3.
Le pourcentage de survie est exprimée en ordonnées.

Une protection partielle au challenge (50%) est observé en présence de C1 (10⁹ Ad-gp50/ml).

Pour C2 (3.10⁸ /ml ) et C3 (3.10⁷ /ml), une absence de protection significative est observée par rapport à un témoin absolu (non vacciné). Le témoin absolu (non vacciné) présente un taux de mortalité de 100%.

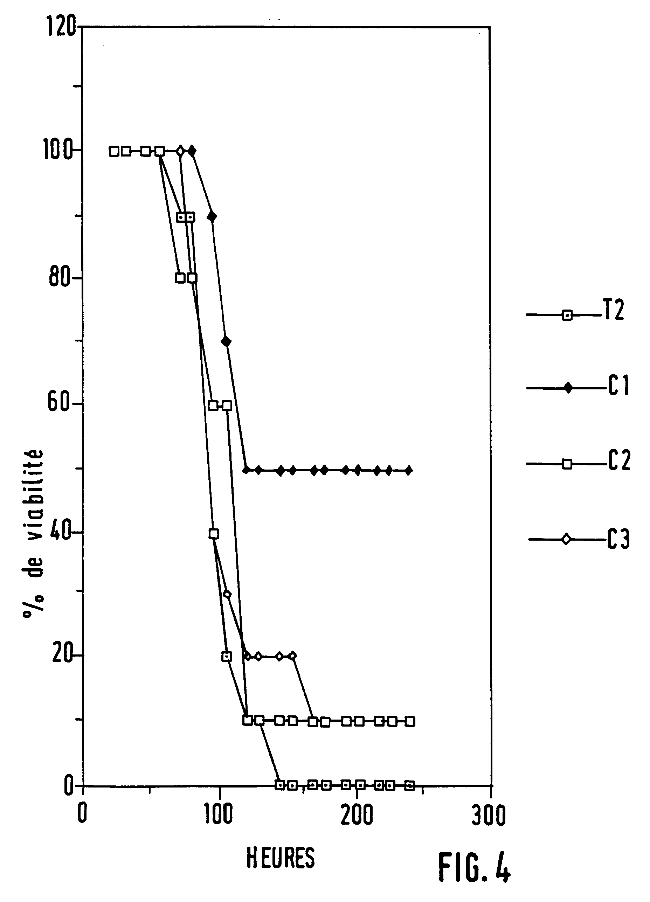

La figure 4 représente la cinétique de survie à l'épreuve de l'infection par le virus Aujeszky après immunisation sans adjuvant.
Le pourcentage de viabilité est exprimé en ordonnée.

On note que la mortalité est observée avant 120 heures pour le témoin comme pour les concentrations C2 et C3.

On remarque donc qu'en l'absence d'adjuvant, l'adénovirus recombinant est incapable d'induire des anticorps anti gp50 et d'induire une quelconque protection au challenge avec le virus Aujeszky infectieux à une concentration inférieure à 10⁹ Ad- gp50 /ml. Seule une protection partielle est obtenue avec une concentration de 10⁹ Ad-gp50/ml.

### - Exemple 2 : Induction d'une réponse immunitaire et d'une protection à l'épreuve du virus Aujeszky en présence d'adjuvant huileux de type E/H, H/E, E/H/E :

Les 6 adjuvants décrits dans le tableau 1 ci-après ont été testés quant à leur potentialité à induire des anticorps anti gp 50 et à induire une résistance après une épreuve avec des virus Aujeszky infectieux telle que définie dans l'exemple 1. Les préparations vaccinales sont réalisées à la seringue par mélange de l'adjuvant et des virus recombinants aux concentrations C1, C2 et C3, telles que définies dans l'exemple 1, dans les préparations suivantes :

### 1) Préparations vaccinales H/E

préparation n°1
   25% adjuvant (huile + système émulgateur)⁽¹⁾
   75% composition immunogène
préparation n°2
   25% adjuvant (huile + système émulgateur + avridine) ⁽²⁾
   75% composition immunogène
préparation n°3
   20% adjuvant (huile + système émulgateur)⁽³⁾
   80% composition immunogène

(1)= Montanide ISA 25
(2)= Montanide ISA 25A
(3) Montanide ISA 28

### 2) Préparations vaccinales E/H/E

préparation n°4
   50% adjuvant (huile + système émulgateur)⁽⁴⁾
   50% composition immunogène

(4) Montanide ISA 206 (4) Montanide ISA 206

### 3 ) Préparations vaccinales E/H

préparation n°5
   55% adjuvant (huile + système émulgateur)⁽⁵⁾
   45% composition immunogène
préparation n°6
   70% adjuvant (huile + système émulgateur)⁽⁶⁾
   30% composition immunogène

(5) Montanide ISA 50
(6) Montanide ISA 708

**TABLEAU I**

| **N°** | **ADJUVANT** | **HUILE** | **TYPE DE VACCIN** | **% Phase aqueuse/ Adjuvant (en poids)** | **VISCOSITE (m.Pa.s)** | **CONDUCTIVITE 25°C (µS. cm**^{**-1**}**)** |
|---|---|---|---|---|---|---|
| 1 | MONTANIDE ISA 25 | Minérale | H/E | 75 % | 20 | 5000 |
| 2 | MONTANIDE ISA 25A | Minérale + Avridine* | H/E | 75 % | 20 | 5000 |
| 3 | MONTANIDE ISA 28 | Minérale + non minérale | H/E | 75 % | 25 | 1000 |
| 4 | MONTANIDE ISA 206 | Minérale | E/H/E | 50 % | 50 | 1000 |
| 5 | MONTANIDE ISA 50 | Minérale | E/H | 50 % | 200 | 1 |
| 6 | MONTANIDE ISA 708 | Non minérale | E/H | 30 % | 70 | 1 |

| | | | | | | |
|---|---|---|---|---|---|---|
| * Avridine = N,N-dioctadecyl-N-bis (2-hydroxyethyl)-propanediamine | | | | | | |

Tous les Montanides ISA mentionnés ci-dessus sont fabriqués et commercialisés par la société SEPPIC. Les émulgateurs qu'ils comportent, sont des dérivés d'ester de mannitol.
Ces préparations sont comparées :
- aux préparations vaccinales sans adjuvant décrites dans l'exemple 1 (T1)
- à deux groupes témoins constitués pour l'un des souris non vaccinées (T2) et pour l'autre de souris dans lesquelles on injecte l'adjuvant, Montanide ISA 50, en absence d'Ad-gp50 (T3).

Les dosages des anticorps anti gp50, les mesures des pourcentages de taux de survie et les mesure de cinétique de mortalité sont réalisés selon les protocoles décrits dans l'exemple 1 ci-dessus.

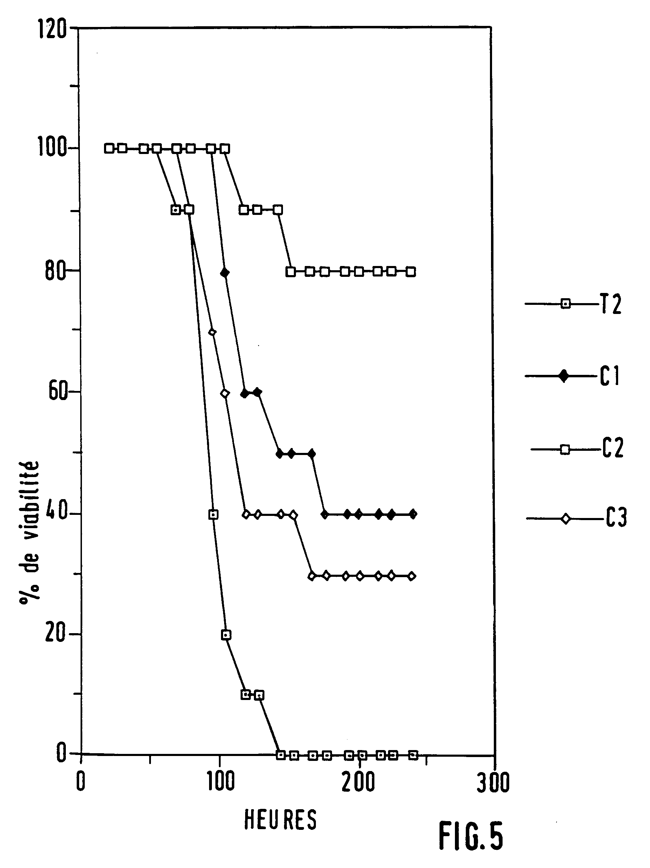

Les résultats obtenus sont résumés dans le tableau 2 ci-dessous et sur les figures 5, 6, 7, 8, 9, 10, dans lesquels C1, C2, C3 ont les mêmes significations que dans l'exemple 1.

**TABLEAU II**

| **ADJUVANT** | **Ad - gp50 concentration** | **ELISA titre** |
|---|---|---|
| Montanide ISA 25 | C1 | > 256 |
| Montanide ISA 25 | C2 | 128 |
| Montanide ISA 25 | C3 | < 4 |
| Montanide ISA 25 A | C1 | > 256 |
| Montanide ISA 25 A | C2 | 90 |
| Montanide ISA 25 A | C3 | 90 |
| Montanide ISA 28 | C1 | > 256 |
| Montanide ISA 28 | C2 | 256 |
| Montanide ISA 28 | C3 | 32 |
| Montanide ISA 206 | C1 | > 256 |
| Montanide ISA 206 | C2 | 256 |
| Montanide ISA 206 | C3 | > 256 |
| Montanide ISA 50 | C1 | > 256 |
| Montanide ISA 50 | C2 | > 256 |
| Montanide ISA 50 | C3 | < 4 |
| Montanide ISA 708 | C1 | > 256 |
| Montanide ISA 708 | C2 | > 256 |
| Montanide ISA 708 | C3 | < 4 |
| néant (T1) | C1 | 256 |
| néant | C2 | < 4 |
| néant | C3 | < 4 |
| Montanide ISA 50 (T3) | 0 | < 4 |
| néant (T2) | 0 | < 4 |

### a) Résultats avec des émulsions type H/E

### - Dosage des anticorps anti gp 50

Le tableau 2 montre que les taux d'anticorps anti gp 50 sont significativement supérieurs à ceux obtenus avec le témoin sans adjuvant et ce respectivement pour les Montanides ISA 25 , Montanide ISA 25 A, qui est le Montanide ISA 25 additionnée d'avridine, stimulant de l'immunité cellulaire et le Montanide ISA 28.

### -Epreuve avec le virus virulent

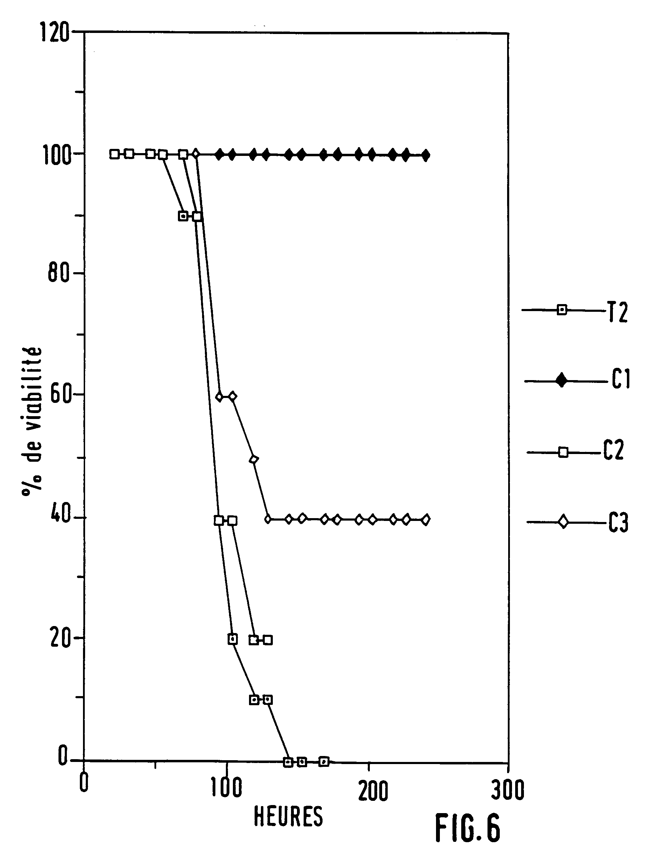

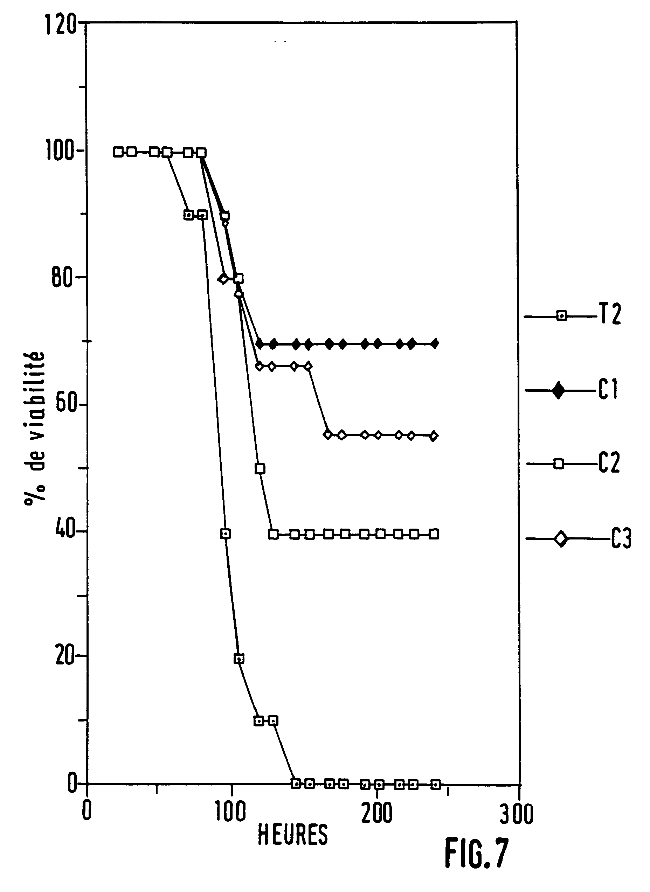

Les figures 5, 6, 7, indiquent le pourcentage de viabilité en fonction du temps pour les trois concentrations vaccinales, comparées au témoin T2, et ce respectivement pour les Montanides ISA 25 (figure 5), Montanide ISA 25 A (figure 6), et le Montanide ISA 28 (figure 7);

L'effet adjuvant est à comparer aux résultats obtenus sans adjuvant et représenté sur la figure 4.

### b) Résultats avec des émulsions de type E/H/E :

### - Dosage des anticorps anti gp 50

Les résultats représentée sur le tableau 2 montrent clairement que le taux d'anticorps anti gp 50 obtenu en présence de Montanide ISA 206 est significativement supérieur à ceux obtenus avec le témoin T2 pour les 3 concentrations de gp50 testés.

### - Epreuve avec le virus virulent

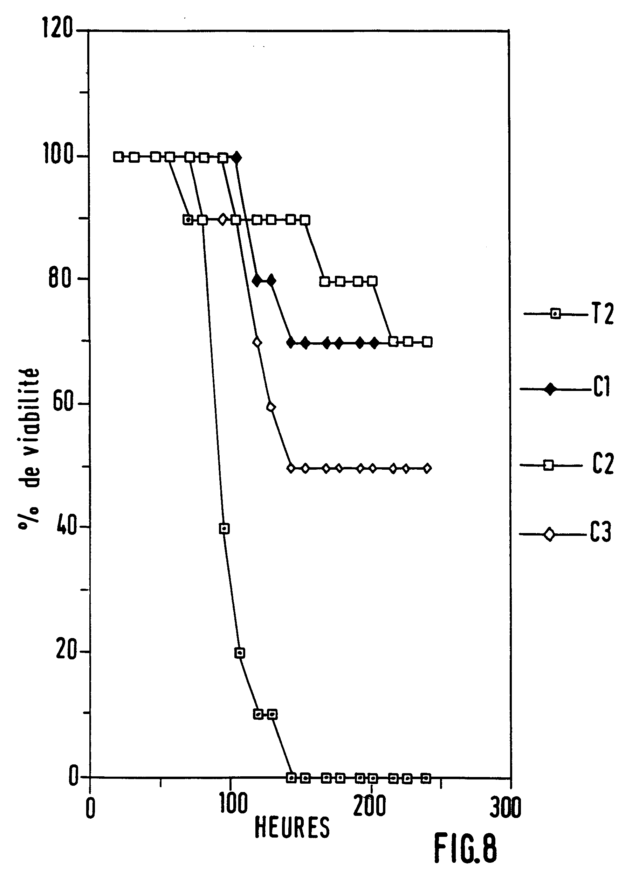

La figure 8 représente les résultats après challenge obtenus avec l'huile minérale Montanide ISA 206 en association avec l'Ad-gp 50.
Un taux de survie de 70% et 50% sont obtenus pour les concentrations les plus faibles d'Ad-gp 50

### c) Résultats avec des émulsions de type E/H

### - Dosage des anticorps anti gp 50

Le tableau 2 montre un taux d'anticorps significativement détectables à la concentration de 3.10⁸ Ad- gp50/ml tandis que pour cette même concentration la détection d'anticorps anti gp 50 est impossible en l'absence d'adjuvant.

### - Epreuve avec le virus virulent

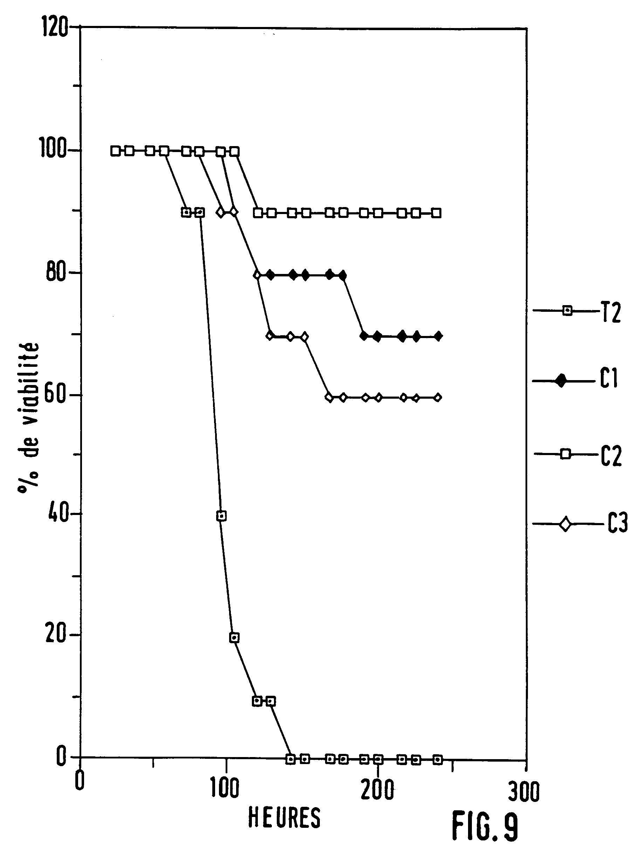

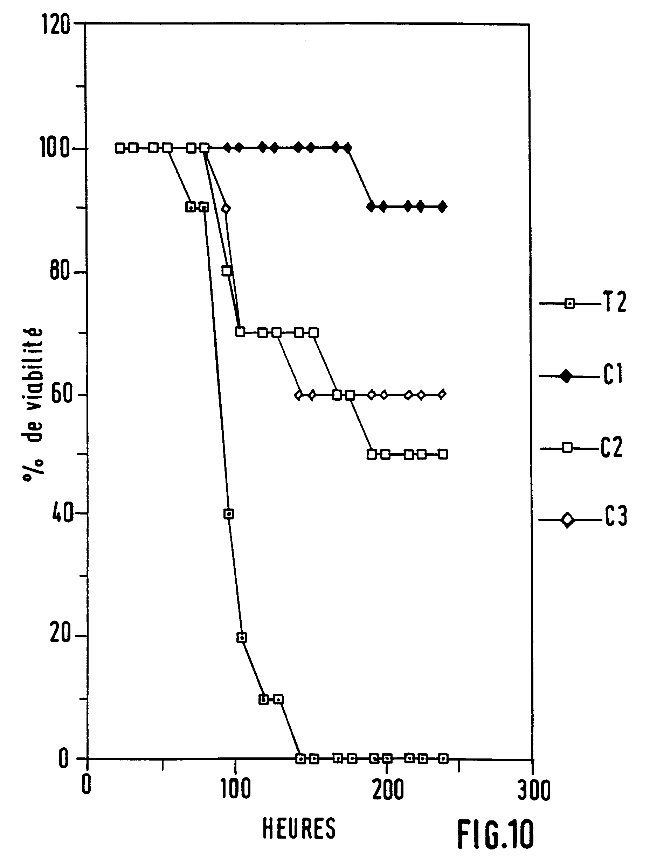

Les résultats obtenus avec le Montanide ISA 50 (huile minérale) et le Montanide ISA 708 (huile minérale) sont représentés respectivement sur la figure 9 et 10.
Un taux de survie supérieur à 60% pour le Montanide ISA 50 et à 50% pour le Montanide ISA 708 est obtenu pour toutes les concentrations d'Ad- gp50.

En l'absence d'adjuvant selon l'invention, on rappelle (voir figure 4) que le taux de survie est de 10% avec les concentrations C2 et C3.

Le tableau 2 indique donc clairement que le taux d'anticorps anti gp 50 en présence des adjuvants selon l'invention est significativement supérieur à celui obtenu en absence d'adjuvant huileux.

Les figures 4 à 10 indiquent que, alors qu'en l'absence d'adjuvant huileux selon l'invention, (figure 4) on observe une protection partielle (50%) au challenge en présence de 10⁹ Ad-gp50/ml et une absence de protection (10%) en présence de 3.10⁸ et 3.10⁷ Ad-gp50/ml.

L'utilisation de Montanide ISA permet d'obtenir des taux de protection jusqu'à 100% pour la concentration la plus élevée (10⁹ Ad-gp50/ml), 90% (3.10⁸ Ad-gp50/ml) et 60% (3.10⁷ Ad-gp50/ml).

L'ensemble de cet étude permet de montrer l'intéret d'utiliser des adjuvants de type huileux selon l'invention sur des vaccins sous-unitaires recombinants vivants permettant d'augmenter de manière significative le taux d'anticorps spécifiques du gène exprimé et la protection contre l'agent infectieux.
Ces résultats significatifs sont obtenus en primovaccination.
Parmi les vaccins adjuvés efficaces testés on distingue donc :
- **Les vaccins de type E/H** (Montanide ISA 50 et ISA 708):
   * ils induisent d'importants taux d'anticorps aux concentrations de 10⁹ et 3.10⁸ Ad-gp50/ml tandis qu'ils ne sont pas détectables à la concentration de 3.10⁷ Ad-gp50/ml.
   * ils protègent contre le challenge avec le virus Aujeszky infectieux pour les trois concentrations testées à des taux supérieurs à 50%.
   Il semble donc ici qu'il n'y a pas ici de stricte corrélation entre le taux de survie et l'induction d'anticorps anti gp 50.
   Ce phénomène pourrait être lié à :
- une induction très importante d'une classe d'immunoglobuline non détectable par le dosage ELISA utilisé (IgM, IgA, IgE).
- et/ou l'induction d'une immunité de type cellulaire.
- **Les vaccins de type E/H/E** (Montanide ISA 206) :
   * ils induisent des taux d'anticorps très importants pour les trois concentrations d'Ad-gp50 testés (10⁹/ml, 3.10⁸/ml, 3.10⁷/ml).
   * ils protègent contre le virus Aujeszky infectieux pour les trois concentrations testées à des taux supérieurs à 50%.
- **Les vaccins de type H/E** avec les Montanide ISA 25 et ISA 28
   * ils induisent des taux d'anticorps spécifiques anti gp 50 importants aux concentrations de 10⁹ et 3.10⁸ Ad-gp50/ml tandis qu'en présence de Montanide ISA 28 on détecte encore la présence d'anticorps anti gp 50 à la concentration d'Ad-gp 50 la plus faible (3.10^{7/ml).}
   * ils protègent contre le virus Aujeszky infectieux avec le Montanide ISA 28 à des taux compris entre 40 et 55%.

Ce type de vaccins (H/E) apparaît cependant globalement moins efficaces que les vaccins de type E/H ou E/H/E.
- **Les vaccins de type H/E avec un adjuvant de l'immunité cellulaire**.
   L'adjonction d'un adjuvant de type avridine au Montanide ISA 25 permet d'obtenir des protections à l'épreuve de 100 % pour la concentration d'Ad-gp50 la plus élevée (10⁹/ml) sans induire une quantité plus importante d'anticorps anti gp 50.
- **Obervations de réactions locales:**
   Une observation macrocospique ne révèle ni de réactions locales, ni de granulomes au site d'injection.

### EXEMPLE 3

### Induction de la synthèse d'anticorps et d'Interleukines

Les 6 adjuvants décrits dans le Tableau 1 ont été testés quant à leur potentialité à induire la synthèse de l'Interleukine IL2.

Les compositions de vaccin comprenant lesdits adjuvants ont été injectées à différents groupes de 10 souris chacun.

Chaque groupe a été vacciné avec une composition de vaccin comprenant l'un des 6 adjuvants et Ad-gp 50 en des concentrations C1, C2 ou C3, telle que définies dans l'exemple 1.

A titre comparatif, on a injecté à d'autres groupes de 10 souris une composition ne comprenant pas, soit un adjuvant selon l'invention, soit Ad-gp 50, soit encore ni adjuvant ni Ad-gp 50.

Dans tous les cas, chaque souris a été traitée par 100 µl de composition.

Le titre sérique en IL2, obtenus 14 jours après la vaccination, figurent dans le Tableau 3, ci-après. Ce titre a été mesuré par une technique ELISA commercialisée par la société Genzyme corps.

**TABLEAU 3**

| **ADJUVANT** | **Ad - gp50 (concentration)** | **IL2 titre (pg/ml)** |
|---|---|---|
| Montanide ISA 25 | C1 | 70 |
| Montanide ISA 25 | C2 | 55 |
| Montanide ISA 25 | C3 | 60 |
| Montanide ISA 25 A | C1 | 110 |
| Montanide ISA 25 A | C2 | 75 |
| Montanide ISA 25 A | C3 | 75 |
| Montanide ISA 28 | C1 | 110 |
| Montanide ISA 28 | C2 | 75 |
| Montanide ISA 28 | C3 | 100 |
| Montanide ISA 206 | C1 | 110 |
| Montanide ISA 206 | C2 | 95 |
| Montanide ISA 206 | C3 | 90 |
| Montanide ISA 50 | C1 | 125 |
| Montanide ISA 50 | C2 | 100 |
| Montanide ISA 50 | C3 | 70 |
| Montanide ISA 708 | C1 | 140 |
| Montanide ISA 708 | C2 | 100 |
| Montanide ISA 708 | C3 | 60 |
| néant | C1 | 55 |
| néant | C2 | < 50 |
| néant | C3 | < 50 |
| Montanide ISA 50 | néant | < 50 |
| néant | néant | < 50 |

Comme il apparaît des résultats obtenus, la synthèse d'IL2 est fortement stimulée par un adjuvant associé à l'Ad-gp 50 , selon l'invention.

### EXEMPLE 4

Les adjuvants Montanide ISA 25, Montanide ISA 206 et Montanide 50, tels que décrits dans le Tableau 1, ont été testés quant à leur potentialité à induire la synthèse de l'Interleukine IL6.

Les compositions de vaccin comprenant lesdits adjuvants ont été injectées à différents groupes de 10 souris chacun.

Chaque groupe a été vacciné avec une composition de vaccin comprenant l'un desdits adjuvants et Ad-gp 50 en des concentations C₁, C₂ ou C₃, telles que définies dans l'exemple 1.

A titre comparatif, on a traité d'autres groupes de 10 souris en leur injectant une composition ne comprenant pas, soit un adjuvant selon l'invention, soit Ad-gp50, soit encore, ni adjuvant, ni Ad-gp 50. Dans tous les cas, on a injecté 100 µl de composition à chaque souris. Le titre sérique en IL6, obtenu 14 jours après l'injection, figure dans le Tableau 4, ci-dessous. Ce titre en IL6 a été mesuré par une technique ELISA classique commercialisée par la société Endogen.

**TABLEAU 4**

| **ADJUVANT** | **Ad-gp50** | **IL6 (pg/ml)** |
|---|---|---|
| MONTANIDE ISA 25 | C1 | < 50 |
| MONTANIDE ISA 25 | C2 | < 50 |
| MONTANIDE ISA 25 | C3 | < 50 |
| MONTANIDE ISA 25A | C1 | < 50 |
| MONTANIDE ISA 25A | C2 | < 50 |
| MONTANIDE ISA 25A | C3 | < 50 |
| MONTANIDE ISA 206 | C1 | 1250 |
| MONTANIDE ISA 206 | C2 | 110 |
| MONTANIDE ISA 206 | C3 | 1250 |
| MONTANIDE ISA 50 | C1 | < 50 |
| MONTANIDE ISA 50 | C2 | < 50 |
| MONTANIDE ISA 50 | C3 | <50 |
| néant | C1 | < 50 |
| néant | C2 | < 50 |
| néant | C3 | <50 |
| MONTANIDE ISA 50 | néant | < 50 |
| ----- | néant | < 50 |

Comme il apparaît des résultats obtenus, la synthèse d'IL6 est stimulée par la composition de vaccin comprenant l'adjuvant Montanide ISA 206, du type émulsion E/H/E, associé à l'Ad-gp50.

### EXEMPLE 5

Les 6 adjuvants décrits dans le Tableau 1 ont été testés quant à leur potentialité à induire la synthèse des quatre différentes sous-classes d'Anticorps anti gp50, à savoir les Ig G1, Ig G2a, Ig G2b et Ig G3.

Les compositions de vaccin ont été injectées à des groupes de 10 souris chacun. Chaque groupe de souris a été vacciné avec une composition de vaccin comprenant l'un des 6 adjuvants et Ad-gp50 en des concentrations C1, C2 ou C3, telles que définies dans l'exemple 1.

A titre comparatif, on a traité d'autres groupes de 10 souris en leur injectant une composition ne comprenant pas, soit un adjuvant selon l'invention, soit Ad-gp 50, soit encore, ni adjuvant, ni Ad-gp 50.

Dans tous les cas, les souris ont chacune été traitées par injections de 100 µl de composition selon l'invention ou comparative.

Le titre sérique (exprimé par la dernière dilution à laquelle l'adsorption est supérieure à celle du témoin consistant en une composition comprenant un adjuvant et pas Ad-gp50), en chaque anticorps IgG, obtenus 14 jours après l'injection de ladite composition, figure dans le Tableau 5, ci-après. Le titre sérique a été mesuré de manière classique par une méthode ELISA.

**TABLEAU 5**

| **ADJUVANT** | **Ad-GP 50** | **IgG1** | **IgG2a** | **IgG2b** | **IgG3** |
|---|---|---|---|---|---|
| Montanide ISA 25 | C1 | 1024 | 32 | 32 | 8 |
| Montanide ISA 25 | C2 | 256 | 32 | 16 | 8 |
| Montanide ISA 25 | C3 | 16 | <8 | <8 | <8 |
| Montanide ISA 25A | C1 | 1024 | 128 | 128 | 64 |
| Montanide ISA 25 A | C2 | 512 | 16 | 16 | 8 |
| Montanide ISA 25 A | C3 | 128 | 16 | 32 | <8 |
| Montanide ISA 28 | C1 | 512 | 128 | 128 | 16 |
| Montanide ISA 28 | C2 | 512 | 64 | 128 | <8 |
| Montanide ISA 28 | C3 | 128 | <8 | <8 | <8 |
| Montanide ISA 206 | C1 | 2048 | 1024 | 512 | 32 |
| Montanide ISA 206 | C2 | 512 | 128 | 64 | 16 |
| Montanide ISA 206 | C3 | 1024 | 128 | 64 | 32 |
| Montanide ISA 50 | C1 | 1024 | 512 | 512 | 128 |
| Montanide ISA 50 | C2 | 512 | 64 | 64 | 64 |
| Montanide ISA 50 | C3 | <8 | <8 | <8 | <8 |
| Montanide ISA 708 | C1 | 5096 | 512 | 512 | 128 |
| Montanide ISA 708 | C2 | 1024 | 64 | 64 | 32 |
| néant | C1 | 128 | 64 | 32 | 8 |
| néant | C2 | 8 | <8 | <8 | <8 |
| néant | C3 | <8 | <8 | <8 | <8 |
| Montanide ISA 50 | néant | <8 | <8 | <8 | <8 |
| néant | néant | <8 | <8 | <8 | <8 |

Comme il apparaît des résultats, une composition de vaccin selon l'invention permet la synthèse des 4 sous-classes d'anticorps anti-gp 50.

Il va de soi que les exemples ci-dessus sont donnés à titre illustratif. L'invention concerne aussi toute composition contenant une ou des huiles minérales, non minérales ou mixtes (minérale + non minérale) associé à un système émulgateur présentant les caractéristiques physicochimiques (viscosité, conductivité) des Montanide ISA et susceptibles d'être émulsionnée avec une solution aqueuse contenant un vaccin recombinant, dans lequel une protéine ou une peptide immunogène de n'importe quel agent pathogène est exprimé dans un vacteur dont la réplication est compatible avec une émulsion huileuse.

## Revendications

1. Composition de vaccin vivant recombinant caractérisée en ce qu'elle comprend au moins un vaccin sous unitaire recombinant vivant associée à une émulsion injectable comportant (i) au moins une phase aqueuse et (ii) une phase huileuse comprenant au moins une huile.

2. Composition selon la revendication 1, caractérisée en ce que le vaccin sous unitaire recombinant vivant comporte un vecteur recombinant consistant en un virus recombinant, de préférence un virus recombinant non enveloppé.

3. Composition selon la revendication 2, caractérisée en ce que le virus recombinant est choisi parmi les adénovirus, le virus de la vaccine, le Canarypox virus, les herpès virus ou les baculovirus.

4. Composition selon l'une des revendications 2 et 3, caractérisée en ce que le virus recombinant est un adénovirus.

5. Composition selon l'une des revendications 2 à 3 caractérisée en ce que le virus recombinant est le canarypox virus.

6. Composition selon l'une des revendications 2 à 3 caractérisée en ce que le virus recombinant est un baculovirus.

7. Composition selon l'une des revendications 1 à 6, caractérisée en ce que la phase huileuse comporte un système émulgateur.

8. Composition selon l'une des revendications 1 à 7 caractérisée en ce que ladite émulsion est du type E/H/E ou du type E/H.

9. Composition de vaccin vivant contre un virus enveloppé ou un micro-organisme pathogène caractérisée par l'association avec une émulsion injectable à base d'eau et d'une phase huileuse comportant au moins une huile et, le cas échéant, un système émulgateur, d'un vaccin sous-unitaire recombinant vivant consistant en un vecteur recombinant viral vivant dont le génome contient, une séquence codant pour une sous unité antigénique induisant une synthèse d'anticorps et/ou un effet protecteur contre le susdit virus enveloppé ou micro-organisme pathogène.

10. Composition selon la revendication 9 et 10 caractérisée en ce que le vecteur viral est un virus non-enveloppé.

11. Composition selon l'une des revendications 9 et 10 caractérisée en ce que ladite séquence code pour une sous-unité antigénique induisant un effet protecteur contre les virus HIV tels les virus HIV1 ou HIV2.

12. Composition selon l'une des revendications 9 et 10 caractérisée en ce que la dite séquence code pour une sous-unité antigénique induisant un effet protecteur contre le virus FIV.

13. Composition selon l'une des revendications 9 et 10, caractérisée en ce que ladite séquence code pour une sous-unité antigénique induisant un effet protecteur contre le virus Aujesky.

14. Composition selon la revendication 13, caractérisée en ce que la séquence contenue dans le génome du vecteur viral est une séquence codant pour la glycoprotéine gp 50 du virus Aujeszky, et en ce qu'il est protecteur vis à vis d'une infection par le virus Aujeszky.

15. Composition selon l'une des revendications 1 à 14 caractérisée en ce que l'huile est de type minéral ou non minéral.

16. Composition selon l'une des revendications 1 à 15, caractérisée en ce qu'elle présente une stabilité supérieure ou égale à 12 mois à 4°C et une viscosité inférieure à 300 millipascals .s à 25°C.

17. Composition selon l'une des revendications 1 à 16, caractérisée par le fait que les proportions pondérales de la phase huileuse contenant de l'huile associée ou non au système émulgateur, et de la phase aqueuse contenant les antigènes ou les principes actifs varient respectivement de 5 à 95% de phase huileuse, pour de 95 à 5% de phase aqueuse notamment de 25 à 75% de phase huileuse pour 75 à 25% de phase aqueuse.

18. Composition selon l'une des revendications 1 à 17 caractérisée en ce que la phase huileuse contient un système émulgateur comprenant (i) un ester obtenu par condensation d'un acide gras, liquide à 20°C, sur un sucre ou du glycérol, ou/et (ii) un dérivé d'un tel ester.

19. Composition selon la revendication 18 caractérisée en ce que ledit sucre est le mannitol.

20. Procédé de préparation d'une composition de vaccin vivant recombinant selon l'une des revendications 1 à 19, caractérisé en ce qu'il consiste à émulsionner un vaccin vivant recombinant en solution aqueuse avec une huile minérale ou non minérale ou un mélange d'huile minérale ou non minérale sous la forme d'une émulsion E/H ou H/E ou E/H/E.

21. Procédé selon la revendication 20, caractérisé en ce que l'huile minérale ou non minérale peut être associé à un adjuvant de l'immunité cellulaire tel l'avridine.

22. Utilisation d'une composition selon l'une des revendications 1 à 19 pour préparer un vaccin destiné à l'homme ou l'animal.

23. Utilisation d'une composition selon l'une des revendications 1 à 19 pour préparer un vaccin destiné à l'induction de la synthèse de cytokines, en particulier des interleukines.

24. Utilisation selon la revendication 23, caractérisée en ce que lesdites interleukines consistent en l'IL2 ou l'IL6.

## Claims

1. Recombinant live vaccine composition characterized in that it comprises at least one live recombinant sub unitary vaccine associated with an injectable emulsion comprising (i) at least one aqueous phase and (ii) one oily phase comprising at least one oil.

2. Composition according to claim 1, characterized in that the live recombinant sub-unitary vaccine comprises a recombinant vector consisting of a recombinant virus, preferably a non-enveloped recombinant virus.

3. Composition according to claim 2, characterized in that the recombinant virus is selected from the adenoviruses, the vaccinia virus, the canarypox virus, the herpes virus or the baculoviruses.

4. Composition according to either of claims 2 or 3, characterized in that the recombinant virus is an adenovirus.

5. Composition according to either of claims 2 or 3, characterized in that the recombinant virus is the canarypox virus.

6. Composition according to either of claims 2 or 3, characterized in that the recombinant virus is a baculovirus.

7. Composition according to one of claims 1 to 6 characterized in that the oily phase comprises an emulsifying system.

8. Composition according to one of claims 1 to 7 characterized in that the said emulsion is of the W/O/W type or the W/O type.

9. Live vaccine composition against an enveloped virus or a pathogenic micro-organism, characterised in that an injectable emulsion based on water and an oily phase comprising at least one oil and, optionally, an emulsifier system, is associated with a live recombinant sub unitary vaccine consisting of a live viral recombinant vector, the genome of which contains a sequence coding for an antigenic sub-unit inducing an antibody synthesis and/or a protective effect against the said enveloped virus or pathogenic micro-organism.

10. Composition according to claim 9 and 10, characterized in that the viral vector is a non-enveloped virus.

11. Composition according to either of claims 9 or 10, characterized in that the said sequence codes for an antigenic sub-unit inducing a protective effect against the HIV viruses such as the HIV1 or HIV2 viruses.

12. Composition according to either of claims 9 or 10, characterized in that the said sequence codes for an antigenic sub-unit inducing a protective effect against the FIV virus.

13. Composition according to either of claims 9 or 10, characterized in that the said sequence codes for an antigenic sub-unit inducing a protective effect against the Aujesky virus.

14. Composition according to claim 13, characterized in that the sequence contained in the viral vector genome is a sequence coding for the glycoprotein gp 50 of the Aujeszky virus and in that it is a protector against infection by the Aujeszky virus.

15. Composition according to one of claims 1 to 14, characterized in that the oil is of the mineral or non mineral type.

16. Composition according to one of claims 1 to 15 characterized in that it has a stability greater than or equal to 12 months at 4°C and a viscosity less than 300 millipascal.s at 25°C.

17. Composition according to one of claims 1 to 16, characterized in that the proportions by weight of the oily phase containing the oil associated or not with the emulsifying system, and the aqueous phase containing antigens or active principles varies respectively from 5 to 95% of oily phase, for 95 to 5% of aqueous phase, in particular 25 to 75% of oily phase for 75 to 25% of aqueous phase.

18. Composition according to one of claims 1 to 17 characterized in that the oily phase contains an emulsifying system comprising (i) an ester obtained by condensation of a fatty acid, liquid at 20°C, with a sugar or glycerol, and/or (ii) a derivative of such an ester.

19. Composition according to claim 18, characterized in that the said sugar is mannitol.

20. Process for the preparation of a recombinant live vaccine composition according to one of claims 1 to 19, characterized in that it consists of emulsifying a recombinant live vaccine in aqueous solution with a mineral oil or non-mineral oil or a mixture of mineral oil or non-mineral oil in the form of a W/O or an O/W or a W/O/W emulsion.

21. Process according to claim 20, characterized in that the mineral oil or non-mineral oil may be associated with a cellular immunity adjuvant such as avridine.

22. Use of a composition according to one of claims 1 to 19, for preparing a vaccine intended for man or animals.

23. Use of a composition according to one of claims 1 to 19, for preparing a vaccine intended for inducing the synthesis of cytokins, in particular interleukins.

24. Use according to claim 23, characterized in that the said interleukins consist of IL2 or IL6.

## Patentansprüche

1. Rekombinante Lebendimpfstoff-Zusammensetzung, gekennzeichnet durch zumindest einen rekombinanten Untereinheits-Lebendimpfstoff in einer injizierbaren Emulsion mit (i) zumindest einer wäßrigen Phase und (ii) einer zumindest ein Öl umfassenden öligen Phase.

2. Zusammensetzung nach Anspruch 1, dadurch gekennzeichnet, daß der rekombinante Untereinheits-Lebendimpfstoff einen in einem rekombinanten Virus und bevorzugt in einem hüllenlosen rekombinanten Virus bestehenden rekombinanten Vektor umfaßt.

3. Zusammensetzung nach Anspruch 2, dadurch gekennzeichnet, daß das rekombinante Virus aus den Adenoviren, den Impfviren, den Canarypox-Viren, den Herpes-Viren oder den Bakuloviren ausgewählt wird.

4. Zusammensetzung nach einem der Ansprüche 2 oder 3, dadurch gekennzeichnet, daß das rekombinante Virus ein Adenovirus ist.

5. Zusammensetzung nach einem der Ansprüche 2 oder 3, dadurch gekennzeichnet, daß das rekombinante Virus der Canarypox-Virus ist.

6. Zusammensetzung nach einem der Ansprüche 2 oder 3, dadurch gekennzeichnet, daß das rekombinante Virus ein Bakulovirus ist.

7. Zusammensetzung nach einem der Ansprüche 1 bis 6, dadurch gekennzeichnet, daß die ölige Phase ein Emulgatorsystem umfaßt.

8. Zusammensetzung nach einem der Ansprüche 1 bis 7, dadurch gekennzeichnet, daß die Emulsionsart E/H/E oder E/H ist.

9. Lebendimpfstoff-Zusammensetzung gegen ein Hüllenvirus oder einen pathogenen Mikroorganismus, dadurch gekennzeichnet, daß einer injizierbaren, auf Wasser und einer zumindest ein Öl umfassenden öligen Phase sowie gegebenenfalls einem Emulgatorsystem basierenden Emulsion ein aus einem rekombinanten, viralen Vektor, dessen Genom eine eine Antigen-Untereinheit codierende Sequenz enthält, die eine Synthese von Antikörpern und/oder eine Schutzwirkung gegen das Hüllenvirus oder den pathogenen Mikroorganismus induziert, bestehender rekombinanter Untereinheits-Lebendimpfstoff beigefügt ist.

10. Zusammensetzung nach Anspruch 9, dadurch gekennzeichnet, daß der virale Vektor ein hüllenloses Virus ist.

11. Zusammensetzung nach einem der Ansprüche 9 oder 10, dadurch gekennzeichnet, daß die Sequenz eine Antigen-Untereinheit codiert, die eine Schutzwirkung gegen HIV-Viren, etwa HIV1 oder HIV2, induziert.

12. Zusammensetzung nach einem der Ansprüche 9 oder 10, dadurch gekennzeichnet, daß die Sequenz eine Antigen-Untereinheit codiert, die eine Schutzwirkung gegen das FIV-Virus induziert.

13. Zusammensetzung nach einem der Ansprüche 9 oder 10, dadurch gekennzeichnet, daß die Sequenz eine Antigen-Untereinheit codiert, die eine Schutzwirkung gegen das Aujeszky-Virus induziert.

14. Zusammensetzung nach Anspruch 13, dadurch gekennzeichnet, daß die in dem Genom des viralen Vektors enthaltene Sequenz eine Sequenz ist, die das Glykoprotein gp 50 des Aujeszky-Virus codiert, und daß sie vor einer Infektion durch das Aujeszky-Virus schützt.

15. Zusammensetzung nach einem der Ansprüche 1 bis 14, dadurch gekennzeichnet, daß das Öl mineralisch oder nicht mineralisch ist.

16. Zusammensetzung nach einem der Ansprüche 1 bis 15, dadurch gekennzeichnet, daß sie bei 4°C genau oder länger als 12 Monate stabil ist und bei 25° eine Viskosität von weniger als 300 mPa·s aufweist.

17. Zusammensetzung nach einem der Ansprüche 1 bis 16, dadurch gekennzeichnet, daß die Gewichtsanteile der dem Emulgatorsystem zugeordnetes oder nicht zugeordnetes Öl enthaltenden öligen Phase und der die Antigene oder die aktiven Stoffe enthaltenden wäßrigen Phase jeweils zwischen 5 und 95% öliger Phase bei 95 bis 5% wäßriger Phase und bevorzugt zwischen 25 bis 75% öliger Phase bei 75 bis 25% wäßriger Phase schwanken.

18. Zusammensetzung nach einem der Ansprüche 1 bis 17, dadurch gekennzeichnet, daß die ölige Phase ein Emulgatorsystem enthält, welches (i) einen durch Kondensation einer bei 20 °C flüssigen Fettsäure auf einen Zucker oder Glyzerol gewonnenen Ester und/oder (ii) ein Derivat eines solchen Esters umfaßt.

19. Zusammensetzung nach Anspruch 18, dadurch gekennzeichnet, daß der Zucker Mannit ist.

20. Verfahren zur Herstellung einer rekombinanten Lebendimpfstoff-Zusammensetzung nach einem der Ansprüche 1 bis 19, gekennzeichnet durch Emulgieren eines rekombinanten Lebendimpfstoffs in wäßriger Lösung mit einem mineralischen oder nicht mineralischen Öl oder einem Gemisch aus mineralischem oder nicht mineralischem Öl in Form einer E/H-, H/E- oder E/H/E-Emulsion.

21. Verfahren nach Anspruch 20, dadurch gekennzeichnet, daß das mineralische oder nicht mineralische Öl einem Zellimmunitäts-Zusatzstoff, etwa Avridin, beigefügt werden kann.

22. Verwendung einer Zusammensetzung nach einem der Ansprüche 1 bis 19 zur Herstellung eines für den Menschen oder für Tiere bestimmten Impfstoffs.

23. Verwendung einer Zusammensetzung nach einem der Ansprüche 1 bis 19 zur Herstellung eines Impfstoffs, der die Synthese von Zytokinen und insbesondere von Interleukinen induziert.

24. Verwendung nach Anspruch 23, dadurch gekennzeichnet, daß die Interleukine aus IL2 oder IL6 bestehen.
